(19) **Europäisches Patentamt European Patent Office Office européen des brevets**

(11) **EP 2 698 113 B1**

(12) **EUROPEAN PATENT SPECIFICATION**

(45) Date of publication and mention
of the grant of the patent:
**19.08.2015 Bulletin 2015/34**

(51) Int Cl.:
*A61B 8/00* (2006.01)          *G01S 7/52* (2006.01)

(21) Application number: **12770545.7**

(22) Date of filing: **13.04.2012**

(86) International application number:
**PCT/KR2012/002800**

(87) International publication number:
**WO 2012/141519 (18.10.2012 Gazette 2012/42)**

(54) **APPARATUS FOR DIAGNOSING ULTRASOUND IMAGE AND METHOD FOR DIAGNOSING SAME**

VORRICHTUNG ZUR DIAGNOSE VON ULTRASCHALLBILDERN UND DIAGNOSEVERFAHREN DAFÜR

APPAREIL DE DIAGNOSTIC D'IMAGE ULTRASON ET PROCÉDÉ DE DIAGNOSTIC ASSOCIÉ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **15.04.2011 KR 20110035207**

(43) Date of publication of application:
**19.02.2014 Bulletin 2014/08**

(73) Proprietor: **Alpinion Medical Systems Co., Ltd.
Hwaseong-si, Gyeonggi-do 445-380 (KR)**

(72) Inventors:
• **LEE, Daehyun**
  **Seoul 138-891 (KR)**
• **CHO, Seongtaek**
  **Seoul 138-931 (KR)**
• **RHO, Sebeom**
  **Seoul 134-022 (KR)**

(74) Representative: **Thornliey, Peter et al
Kilburn & Strode LLP
20 Red Lion Street
London WC1R 4PJ (GB)**

(56) References cited:
JP-B- H 074 385          JP-B2- H 074 385
KR-A- 20030 015 576      US-A- 5 065 396
US-A1- 2010 022 883

• LEQUAN ZHANG ET AL: "Design of a 64 channel
analog receive beamformer for high frequency
linear arrays", ULTRASONICS SYMPOSIUM
(IUS), 2010 IEEE, IEEE, 11 October 2010
(2010-10-11), pages 1968-1971, XP031953050,
DOI: 10.1109/ULTSYM.2010.5935915 ISBN:
978-1-4577-0382-9

**Description**

[Technical Field]

The present disclosure in some embodiments relates to an ultrasonic diagnostic apparatus and an ultrasonic diagnostic method therefor. More particularly, the present disclosure relates to an ultrasound diagnostic imaging apparatus for improving the picture quality of an ultrasonic image effectively by removing spike voltages generated by switching operations between a first and a second applied voltage to a high voltage multiplexer (HVMUX), and an ultrasound diagnostic imaging method therefor.

[Background]

The statements in this section merely provide background information related to the present disclosure and may not constitute prior art.

Rapid developments of the electronics, signal processing and especially, digital signal processing technology have made a significant impact on the field of diagnostic imaging. Diagnostic imaging system, which can see through the internal organs of the human body without incision of the body, is considered the center of medical diagnostic apparatus and instruments. The diagnostic imaging systems in current use include an X-ray diagnostic apparatus, a Magnetic Resonance Imaging (MRI) diagnostic apparatus, an ultrasound diagnostic apparatus and the like, and each has its own merit and demerit. The ultrasound diagnostic imaging apparatus has advantages of a real time diagnosis and better affordability at the price of reduced resolution. As a result, the ultrasound diagnostic imaging apparatus has become one of the essential medical equipment in virtually all the medical areas such as internal medicine, obstetrics and gynecology, pediatrics, urology, ophthalmology, radiology and the like.

The ultrasound wave for the ultrasound diagnostic imaging apparatus uses frequencies ranging from several MHz to tens of MHz. As the ultrasound wave passes through the human body, its speed and the degree of attenuation vary according to a medium. An ultrasound image is basically formed from reflected waves generated by a boundary between different media.

When a speed of the ultrasound passing through the human body is V, a density of the medium is p, and a volume modulus is B, V can be given by Equation 1.

$$V = \sqrt{\frac{B}{\rho}}$$

Equation 1

The speed of the ultrasound wave within the human body varies depending on medium. Researches have been conducted on compensation for variations of the speed of ultrasound wave within the human body.

An important physical property of the ultrasound wave is an attenuation while it is propagated within a medium. Intensity I of the ultrasound wave according to propagation distance z is expressed by Equation 2.

$$I = I_O \exp(-2\alpha z)$$

Equation 2

In equation 2, $\alpha$ denotes an attenuation coefficient. The attenuation coefficient is closely related to frequency of the ultrasound wave. The attenuation coefficient increases nearly linearly in proportion to frequency in a frequency band used for the ultrasound diagnostic imaging apparatus.

Another important property of the ultrasound diagnostic imaging apparatus is a reflection. The reflection relates to a characteristic impedance Z of the medium. Characteristic impedance Z of the medium is expressed by a product of material density $\rho$ and ultrasound speed V as shown in Equation 3.

$$Z = \rho V$$

<div align="right">Equation 3</div>

When the ultrasound is incident to a material having characteristic impedance Z2 from a material having characteristic impedance Z1, reflectivity R is expressed by Equation 4.

$$R = \frac{Z2 - Z1}{Z2 + Z1}$$

<div align="right">Equation 4</div>

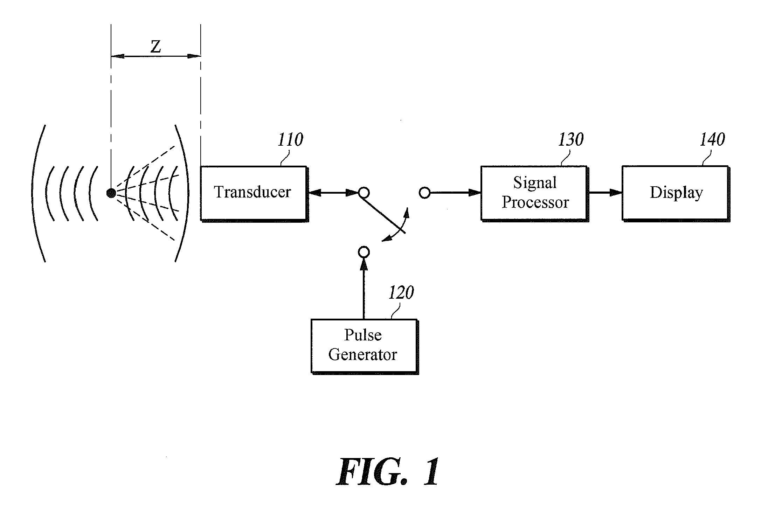

FIG. 1 is a diagram for illustrating a basic principle of an ultrasound diagnostic imaging apparatus. A transducer 110 converts a pulse of an electrical signal generated by a pulse generator 120 into an ultrasound signal and transmits the ultrasound signal to an object. The transducer 110 converts the ultrasound signal reflected and returned from an interface between different media to the corresponding electrical signal and transmits the same to a signal processor 130. The signal processor 130 performs signal processings for the signal received from the transducer 110, such as a time gain compensation (TGC) amplification, a Rx beamforming, an echo processing, a spectral doppler processor (SDP)/color doppler processor (CDP), a digital scan converter (DSC) and the like, and then the processed signal is displayed as an image on a display 140.

Meanwhile, the number of channels through which the pulse generator 120 sends pulses, affects the quality of the ultrasound image such as focusing of an aperture. Taking this in consideration and in order to offer more elements, a TI (transducer interface) board of the ultrasound diagnostic imaging apparatus expands the number of channels toward more elements by using the HVMUX (high voltage multiplexer).

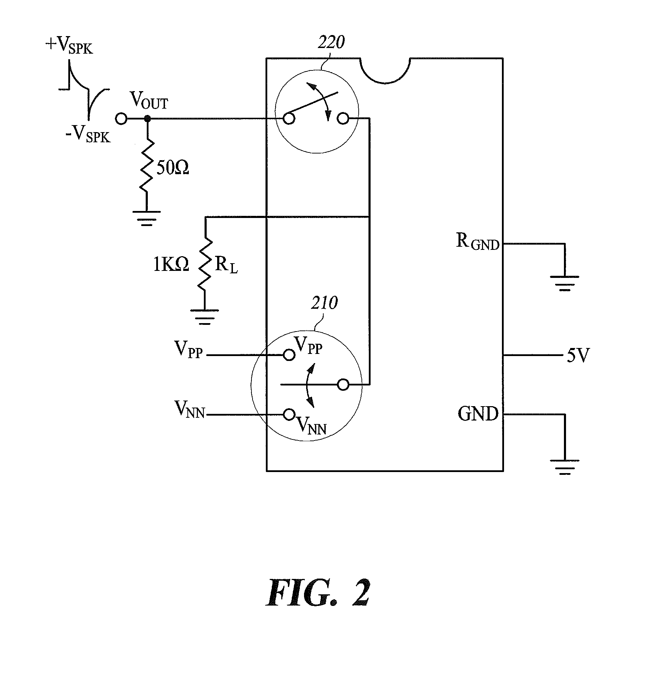

As illustrated in FIG. 2, one channel of the HVMUX is supplied with powers from a first power source $V_{PP}$ and a second power source $V_{NN}$. An electrical signal is output to an output terminal $V_{OUT}$ as a result of a first switching operation 210 of selecting the first power source $V_{PP}$ or the second power source $V_{NN}$ and a second switching operation 220 for determining whether to select a channel.

However, with a typical HVMUX, a spike voltage or a surge voltage as illustrated in FIG. 2 can be generated at the output terminal $V_{OUT}$ by the influence of the bias voltage $V_{PP}$ or $V_{NN}$ simultaneously of the first switching operation 210, which causes to present an unwanted extra stray echo signal in the ultrasound image, in addition to the echo signal of the pulse duly generated by the pulse generator 120.

A device having a 64-channel analog receive beamformer is described in "Design of a 64 Channel Receive Beamformer for High Frequency Linear Arrays" by Lequan Zhang et al, published at pages 1968 to 1971 of the 2010 IEEE Ultrasonics Symposium (IUS). Another multiplexed ultrasound apparatus is described in United States Patent Application Publication US2002/0022883.

[Disclosure]

[Technical Problem]

The embodiments of the present disclosure provide an ultrasound diagnostic imaging apparatus for effectively improving the picture quality of an ultrasonic image by removing spike voltages generated by switching operations between a first and a second applied voltages to a high voltage multiplexer (HVMUX), and an ultrasound diagnostic imaging method therefor.

[Summary]

An aspect of the present disclosure provides an ultrasound diagnostic imaging apparatus, comprising: a pulse generator, a transducer, a multiplexer (MUX) and a dummy MUX. The pulse generator is configured to generate a pulse of an electric signal. The transducer is configured to convert the pulse of the electric signal generated by the pulse generator into an ultrasonic wave signal and to transmit the ultrasonic wave signal to an object. The multiplexer (MUX) is configured to select one or more channels corresponding to at least one element of the transducer, to perform a switching operation between different applied voltages and to enable the pulse of the electrical signal generated by the pulse generator to be transmitted through the selected channel. And the dummy MUX is configured to apply, to an output end of the MUX, a reverse signal of an output signal of the MUX by performing an inverse switching operation which is opposite to the switching operation by the MUX, when the MUX performs the switching operation between the different applied voltages.

The dummy MUX may block the reverse signal applied to the output end of the MUX upon completion of the switching operation between the different applied voltages.

In addition, the MUX may perform the switching operation between applied voltages of +100 V and -100 V.

The dummy MUX may perform the inverse switching operation from - 100 V to +100 V when the switching operation is performed from +100 V to - 100 V for the selected channel.

In addition, the dummy MUX may perform the inverse switching operation from +100 V to -100 V when the switching operation is performed from -100 V to +100 V for the selected channel.

Another aspect of the present disclosure provides an ultrasound diagnostic imagining method, comprising: generating a pulse of an electric signal; performing, by a multiplexer (MUX), a selection of one or more channels corresponding to at least one element of the transducer and a switching operation between different applied voltages; applying, by a dummy MUX, to an output end of the MUX, a reverse signal of an output signal of the MUX by performing an inverse switching operation which is opposite to the switching operation by the MUX; and converting the pulse of the electric signal outputted through the selected channel into an ultrasonic wave signal and transmitting the ultrasonic wave signal to an object.

The applying of the reverse signal may comprise blocking the application of the reverse signal to the output end of the MUX upon completion of the switching operation between the different applied voltages.

In addition, the performing of the channel selection and the switching operation may comprise performing the switching operation between applied voltages of +100 V and -100 V.

In addition, the applying of the reverse signal may comprise performing a voltage application by an inverse switching operation from -100 V to +100 V when the switching operation is performed for the selected channel from +100 V to -100 V.

In addition, the applying of the reverse signal may comprise performing a voltage application by an inverse switching operation from +100 V to -100 V when the switching operation is performed for the selected channel from -100 V to +100 V.

[Advantageous Effects]

According to some embodiments of the present disclosure, an ultrasonic image can be improved in picture quality by removing spike voltages generated by switching operations between a first and a second applied voltage to a HVMUX.

[Description of Drawings]

FIG. 1 is a diagram for illustrating the basic principle of an ultrasound diagnostic imaging apparatus.
FIG. 2 is an exemplary diagram of spike voltages generated in a HVMUX.
FIG. 3 is a schematic diagram of an ultrasound diagnostic imagining apparatus according to at least one embodiment.
FIG. 4 is an exemplary diagram of a pulse generated by a pulse generator in FIG. 3.
FIG. 5 is an exemplary diagram of a MUX and a dummy MUX used in the ultrasound diagnostic imaging apparatus of FIG. 3.
FIG. 6 is a schematic diagram of an ultrasound diagnostic imaging apparatus according to a comparative example.
FIG. 7 is a flowchart of an ultrasound diagnostic imaging method according to at least one embodiment.
FIG. 8 is a flowchart of an ultrasound diagnostic imaging method according to a comparative example.

[Detailed Description]

Hereinafter, some embodiments of the present disclosure will be described in detail with reference to the accompanying drawings. In the following description, like reference numerals designate like elements although the elements are shown in different drawings. Further, in the following description of the embodiments, a detailed description of known functions and configurations incorporated herein will be omitted for the purpose of clarity and for brevity.

Additionally, in describing the components of the present disclosure, terms like first, second, A, B, (a), and (b) are used. These are solely for the purpose of differentiating one component from another, and one of ordinary skill would understand the terms are not to imply or suggest the substances, order or sequence of the components. If a component is described as 'connected', 'coupled', or 'linked' to another component, one of ordinary skill in the art would understand the components are not necessarily directly 'connected', 'coupled', or 'linked' but also are indirectly 'connected', 'coupled', or 'linked' via a third component.

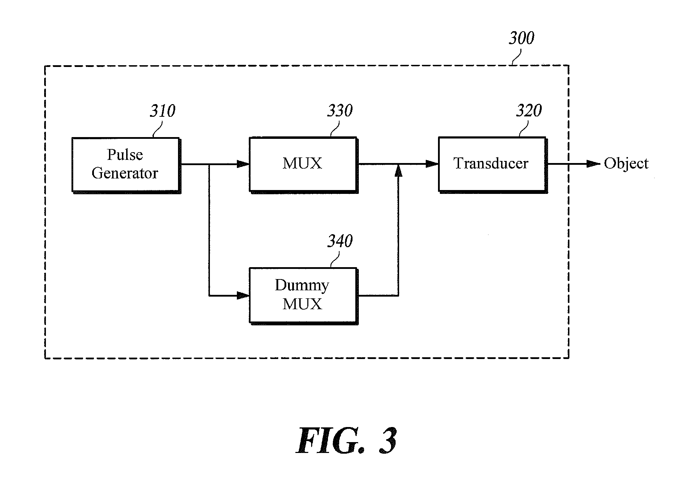

FIG. 3 is an exemplary schematic diagram of an ultrasound diagnostic imaging apparatus according to at least one embodiment of the present disclosure.

Referring to FIG. 3, an ultrasound diagnostic imaging apparatus 300 according to an embodiment of the present disclosure may include a pulse generator 310, a transducer 320, a MUX 330, and a dummy MUX 340.

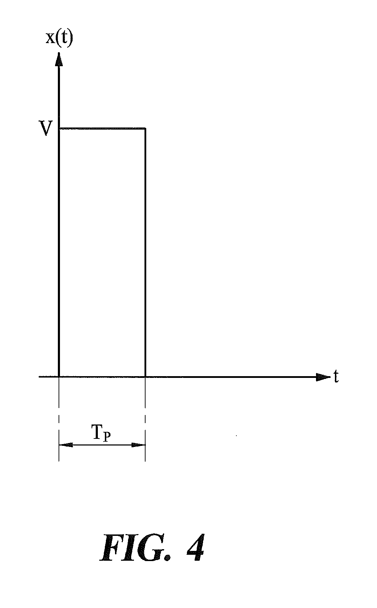

The pulse generator 310 generates a pulse of an electrical signal. The pulse generator 310 excites the transducer 320 with pulse width Tp scaled down as illustrated in FIG. 4 for increasing the axial resolution of the pulse and with pulse size V sufficiently enlarged for increasing the signal/noise (S/N) ratio.

The transducer 320 converts the electrical signal pulse generated by the pulse generator 310 to an ultrasound signal and transmits the ultrasound signal to an object, and converts the ultrasound signal reflected and returned from an interface between different media of the object to an electrical signal and transmits the electrical signal to a signal processor (not shown). Although the instant embodiment also involves the signal processor for processing signals by, for example, the time gain compensation (TGC) amplification, Rx beamforming, echo processing, spectral doppler processor (SDP)/color doppler processor (CDP) or digital scan converter (DSC) and involves displaying images on the display, descriptions of the signal processor and the display will be hereinafter omitted to focus on the subject matter of the present disclosure.

When an ultrasound signal is shot to the object by the transducer 320, if an interface between materials of different acoustic impedances exists in the propagation medium, a reflection occurs at the interface and a part of the ultrasound signal passes through the interface. When there is a plurality of interfaces, the ultrasonic echo signals are sequentially reflected and returned.

In this event, the returned echo applies a stress to the piezoelectric porcelain of the transducer 320, generates an electric field in proportion to the intensity of the echo, and converts the electric field to an electrical signal. One ultrasound pulse transmitted to the object generates a pulse echo from each of the points of various depths (interfaces) within the object, and the echo from a tissue at distance x occurs on a time axis at $t=2x/c$ ($c=1530$ m/s: average sonic speed) in consideration of a pulse reciprocating propagation distance. Accordingly, the reflection position can be determined back from the delay time of the transmitted pulse.

In general, a probe is formed by combining a plurality of elements of the transducer 320, where the MUX 330 selects channels corresponding to the elements of the transducer 320. In this event, different bias voltages are applied to the MUX 330, and the MUX 330 performs a switching operation between the applied different bias voltages and enables the pulse of the electrical signal generated by the pulse generator 310 to be transmitted through the selected channel.

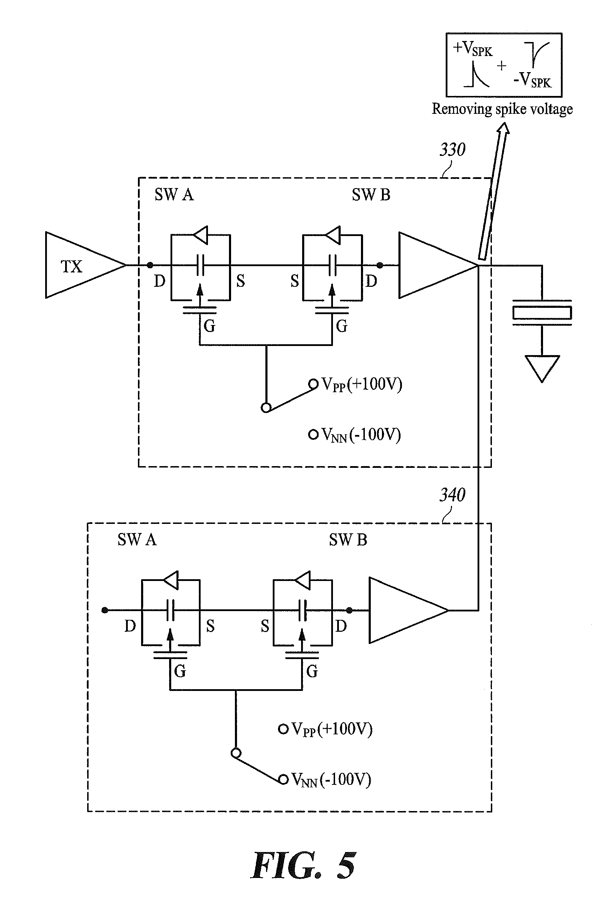

In the switching operation between the different voltages applied to the MUX 330, the dummy MUX 340 applies a reverse signal of the output signal of the MUX 330 to an output terminal of the MUX 330 by performing the inverse switching operation which is opposite to the switching operation of the MUX 330. In order to implement such an operation, the dummy MUX 340 may be configured as illustrated in FIG. 5. Two different voltages $V_{PP}$ and $V_{NN}$ may be applied to one channel selected by the MUX 330 with switching operations performed between $V_{PP}$ and $V_{NN}$. Then, the dummy MUX 340 may have the same configuration as that of the MUX 330 and apply the reverse signal of the output signal of the MUX 330 to the output terminal of the MUX 330 by the switching operation opposite to the switching operation of the MUX 330. For example, the MUX 330 and dummy MUX 340 can be implemented so that the MUX 330 performs the switching operation from applied voltage $V_{PP}$ to applied voltage $V_{NN}$, and in response, the dummy MUX 340 performs the opposite switching operation from $V_{NN}$ to $V_{PP}$ and that an output signal of the dummy MUX 340 is combined with the output signal of the MUX 330. Similarly, when the MUX 330 performs the switching operation from applied voltage $V_{NN}$ to applied voltage $V_{PP}$, the dummy MUX 340 performs the opposite switching operation from $V_{PP}$ to $V_{NN}$, and the output signal of the dummy MUX 340 may be combined with the output signal of the MUX 330. The MUX 330 may have applied voltages of +100 V and -100 V. When the MUX 330 makes the voltage switching from +100 V to -100 V, the dummy MUX 340 performs switching from -100 V to +100 V, and the output signal of the MUX 330 may combine with the output signal of the dummy MUX 340. Likewise, when the MUX 330 performs the voltage switching from -100 V to +100 V, the dummy MUX 340 performs the switching from + 100 V to -100 V and the output signal of the MUX 330 may combine with the output signal of the dummy MUX 340. The applied voltages described herein are only an example, and the switching operations can be performed between various applied voltages.

Meanwhile, the dummy MUX 340 blocks the application of the reverse signal to the output terminal of the MUX 330 upon completing the switching operations of the MUX 330 between the different voltages. This is to block only spike voltages generated in the voltage switching operations of the MUX 330 and to prevent influence by the voltages applied to the dummy MUX 340 after the voltage switching operations of the MUX 330.

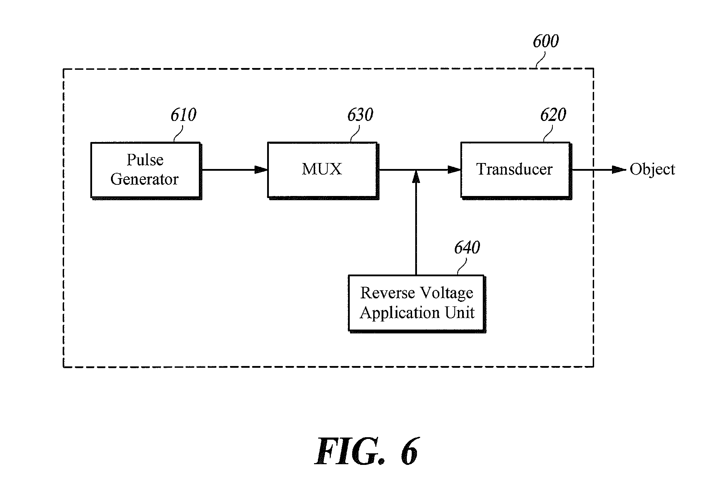

FIG. 6 is a schematic diagram of an ultrasound diagnostic imaging apparatus according to a comparative example.

Referring to FIG. 6, an ultrasound diagnostic imaging apparatus 600 may include a pulse generator 610, a MUX 620, a transducer 630, and a reverse voltage application unit 640. Here, detailed descriptions of the pulse generator 610, the MUX 620 and the transducer 630 will be omitted herein since their configurations and operations are the same as those of the pulse generator 310, the MUX 320 and the transducer 330.

When the switching operation is performed by the MUX 630, the inverse voltage application unit 640 applies a reverse voltage of the spike voltage by the switching operation of the MUX 630 to an output terminal of the selected channel. In this event, the reverse voltage application unit 640 may empirically acquire the spike voltage generated from the switching operation of the MUX 630, and an average value of the empirically acquired spike voltages or a maximum value and a minimum value of the empirically acquired spike voltages are stored.

In response to the switching operation between the different voltages applied to the MUX 630, the reverse voltage application unit 640 forms a virtual spike voltage based on the average value of the empirically acquired spike voltages or the maximum value and the minimum value of the empirically acquired spike voltages, and reverses and then applies the formed spike voltage to the output terminal of the MUX 630.

The reverse signal application unit 640 applies the reverse voltage of the spike voltage simultaneously of the switching operation of the MUX 630, blocking the application of the reverse voltage upon completion of the switching operation from one voltage to another voltage. This blocks or minimizes only the spike voltage generated from the voltage switching operation of the MUX 630 and saves the MUX 630 after its voltage switching operations from being influenced by the reverse voltage applied by the reverse voltage application unit 640.

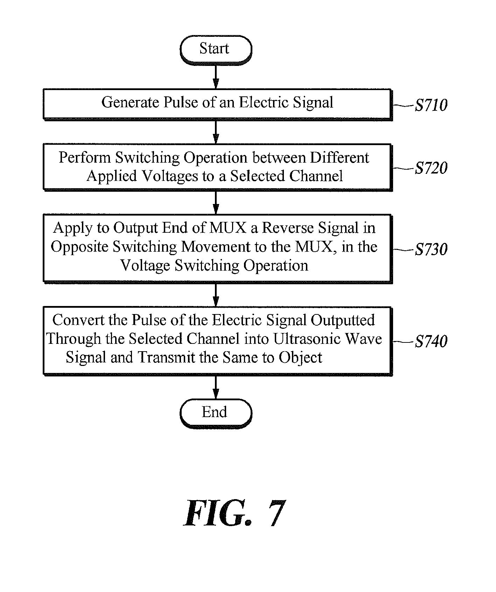

FIG. 7 is a flowchart illustrating an example of an ultrasound diagnostic imaging method according to at least one embodiment of the present disclosure.

Referring to FIGs. 3 and 7, the pulse generator 310 generates a pulse of an electrical signal in step S710. The pulse generator 310 excites the transducer 320 with pulse width Tp scaled down as illustrated in FIG. 4 for increasing the axial resolution of the pulse and with pulse size V sufficiently enlarged for increasing the signal/noise (S/N) ratio.

The MUX 330 performs a switching operation between different applied voltages thereto and enables the pulse of the electrical signal generated by the pulse generator 310 to be transmitted through a selected channel in step S720.

The dummy MUX 340 has the same configuration as that of the MUX 330 and is responsive to the switching operation between the voltages to the MUX 330 for performing an opposite switching operation to the switching operation of the MUX 330 to apply a reverse signal of an output signal of the MUX 330 to an output terminal of the MUX 330 in step S730. For example, when the MUX 330 makes the voltage switching from +100 V to -100 V, the dummy MUX 340 performs switching from -100 V to +100 V, and the output signal of the MUX 330 may combine with the output signal of the dummy MUX 340. Similarly, when the MUX 330 performs the voltage switching from -100 V to +100 V, the dummy MUX 340 performs switching from + 100 V to -100 V and the output signal of the MUX 330 may combine with the output signal of the dummy MUX 340. In this event, the dummy MUX 340 blocks the application of the signal to the output terminal of the MUX 330 upon completion of the switching operation of the MUX 330 between the different voltages.

The transducer 320 converts the pulse of the electrical signal generated by the pulse generator 310 to an ultrasound signal and transmits the ultrasound signal to the object in step S740. In addition, the transducer 320 converts the ultrasound signal reflected and returned from an interface between different media to the corresponding electrical signal and transmits the same to the signal processor. Before the transducer 320, the spike voltage from the switching operation of the MUX 330 is minimized by the signal applied from the dummy MUX 340 to the output terminal of the MUX 330, and accordingly, only the pulse of the electrical signal generated by the pulse generator 310 remains to be presented for the signal conversion.

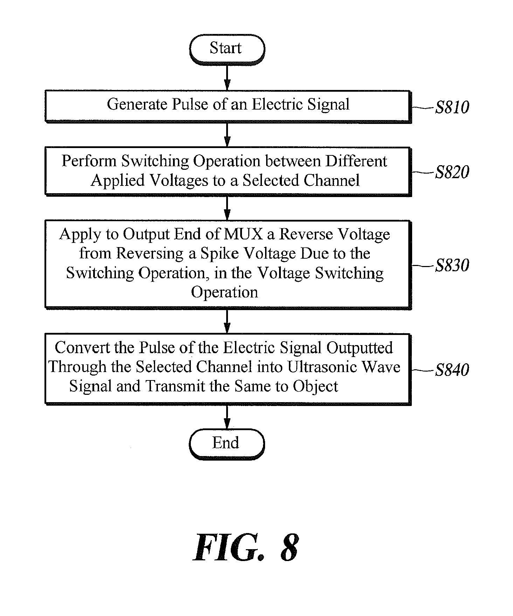

FIG. 8 is a flowchart illustrating a comparative example of an ultrasound diagnostic imaging method.

Referring to FIGs. 6 and 8, the pulse generator 610 generates a pulse of an electrical signal in step S810. The pulse generator 610 excites the transducer 620 with pulse width Tp reduced as illustrated in FIG. 4 for increasing the axial resolution of the pulse and with pulse size V sufficiently enlarged for increasing the signal/noise (S/N) ratio.

The MUX 630 performs a switching operation between different applied voltages and enables the pulse of the electrical signal generated by the pulse generator 610 to be transmitted through a selected channel in step S820.

When the switching operation is performed between the different voltages applied to the MUX 630, the reverse voltage application unit 640 may form a virtual spike voltage based on an average value of spike voltages acquired empirically or a maximum value and a minimum value of the spike voltages acquired empirically in step S830. Further, the step reverses the formed spike voltage, and applies the reversed spike voltage to the output terminal of the MUX 630, in response to the switching operation of the MUX 630. The reverse voltage of the spike voltage described herein is only an example, and the reverse voltage may be formed in variously modified ways.

The transducer 620 converts the pulse of the electrical signal generated by the pulse generator 610 to the ultrasound signal and transmits the ultrasound signal to an object, and converts the ultrasound signal reflected and returned from an interface between different media to the corresponding electrical signal and transmits the same to the signal processor in step S840. Before the transducer 620, the spike voltage from the switching operation of the MUX 630 is minimized by the signal applied from the dummy MUX 640 to the output terminal of the MUX 630, and accordingly, only the pulse of the electrical signal generated by the pulse generator 610 remains to be presented for the signal conversion.

**Claims**

1. An ultrasound diagnostic imaging apparatus, comprising:

   a pulse generator (310) configured to generate a pulse of an electric signal;
   a transducer (320) configured to convert the pulse of the electric signal generated by the pulse generator (310) into an ultrasonic wave signal and to transmit the ultrasonic wave signal to an object;
   a multiplexer, MUX, (330) configured to select one or more channels corresponding to at least one element of the transducer (320), to perform a switching operation between different applied voltages and to enable the pulse of the electrical signal generated by the pulse generator (310) to be transmitted through the selected channel;
   **characterised in that** the ultrasound diagnostic imaging apparatus further comprises
   a dummy MUX (340) configured to apply, to an output end of the MUX (330), a reverse signal of an output signal of the MUX (330) by performing an inverse switching operation which is opposite to the switching operation by the MUX (330), when the MUX (330) performs the switching operation between the different applied voltages.

2. The ultrasound diagnostic imaging apparatus of claim 1, wherein the dummy MUX (340) blocks the reverse signal applied to the output end of the MUX (330) upon completion of the switching operation between the different applied voltages.

3. The ultrasound diagnostic imaging apparatus of claim 1, wherein the MUX (330) performs the switching operation between applied voltages of +100 V and -100 V.

4. The ultrasound diagnostic imaging apparatus of claim 3, wherein the dummy MUX (340) performs the inverse switching operation from -100 V to +100 V when the switching operation is performed from +100 V to -100 V for the selected channel.

5. The ultrasound diagnostic imaging apparatus of claim 3, wherein the dummy MUX (340) performs the inverse switching operation from +100 V to - 100 V when the switching operation is performed from -100 V to +100 V for the selected channel.

6. An ultrasound diagnostic imaging method, comprising:

   generating a pulse of an electric signal;
   performing, by a multiplexer, MUX, (330), a selection of one or more channels corresponding to at least one element of the transducer (320) and a switching operation between different applied voltages;
   applying, by a dummy MUX (340), to an output end of the MUX (330), a reverse signal of an output signal of the MUX (330) by performing an inverse switching operation which is opposite to the switching operation by the MUX (330); and
   converting the pulse of the electric signal outputted through the selected channel into an ultrasonic wave signal and transmitting the ultrasonic wave signal to an object.

7. The ultrasound diagnostic imaging method of claim 6, wherein the applying of the reverse signal comprises blocking the application of the reverse signal to the output end of the MUX upon completion of the switching operation between the different applied voltages.

**Patentansprüche**

1. Diagnostische Ultraschall-Bildgebungsvorrichtung, umfassend:

   einen Impulsgenerator (310), der konfiguriert ist, einen Impuls eines elektrischen Signals zu erzeugen;
   einen Wandler (320), der konfiguriert ist, den Impuls des von dem Impulsgenerator (310) erzeugten elektrischen Signals in ein Ultraschallwellensignal umzuwandeln und das Ultraschallwellensignal an ein Objekt zu übermitteln;

einen Multiplexer (MUX) (330), der konfiguriert ist, einen oder mehrere Kanäle auszuwählen, die mindestens einem Element des Wandlers (320) entsprechen und einen Schaltvorgang zwischen verschiedenen angelegten Spannungen durchzuführen; und es dem Impuls des durch den Impulsgenerator (310) erzeugten elektrischen Signals zu ermöglichen, durch den gewählten Kanal übertragen zu werden;

**dadurch gekennzeichnet, dass** die diagnostische Ultraschall-Bildgebungsvorrichtung ferner umfasst einen Dummy-MUX (340), der konfiguriert ist, an einem Ausgangsende des MUX (330) ein Umkehrsignal von einem Ausgangssignal des MUX (330) durch Ausführen eines inversen Schaltvorgangs, der gegenüber dem Schaltvorgang durch den MUX (330) entgegengesetzt ist, anzulegen, wenn der MUX (330) den Schaltvorgang zwischen den verschiedenen angelegten Spannungen durchführt.

2. Diagnostische Ultraschall-Bildgebungsvorrichtung nach Anspruch 1, wobei der Dummy-MUX (340) das Umkehrsignal, das an das Ausgangsende des MUX (330) angelegt wurde, nach Ausführung des Schaltvorgangs zwischen den verschiedenen angelegten Spannungen blockiert.

3. Diagnostische Ultraschall-Bildgebungsvorrichtung nach Anspruch 1, wobei der MUX (330) den Schaltvorgang zwischen angelegten Spannungen von +100 V und -100 V durchführt.

4. Diagnostische Ultraschall-Bildgebungsvorrichtung nach Anspruch 3, wobei der Dummy-MUX (340) den inversen Schaltvorgang von -100 V nach +100 V durchführt, wenn der Schaltvorgang von +100 V nach -100 V für den gewählten Kanal durchgeführt wird.

5. Diagnostische Ultraschall-Bildgebungsvorrichtung nach Anspruch 3, wobei der Dummy-MUX (340) den inversen Schaltvorgang von +100 V nach -100 V durchführt, wenn der Schaltvorgang von -100 V nach +100 V für den gewählten Kanal durchgeführt wird.

6. Diagnostische Ultraschall-Bildgebungsvorrichtung, umfassend:

Erzeugen eines Impulses eines elektrischen Signals;
Durchführen, durch einen Multiplexer, (MUX), (300), einer Auswahl von einem oder mehreren Kanälen, die mindestens einem Element des Wandlers (320) entsprechen, und eines Schaltvorgangs zwischen verschiedenen angelegten Spannungen;
Anlegen, durch einen Dummy-MUX (340), eines Umkehrsignals von einem Ausgangssignal des MUX (330) an einem Ausgangsende des MUX (330) durch Ausführen eines inversen Schaltvorgangs, der entgegengesetzt zu dem Schaltvorgang durch den MUX (330) ist; und
Umwandeln des Impulses des elektrischen Signals, der durch den gewählten Kanal ausgegeben wird, in ein Ultraschallwellensignal und Übertragen des Ultraschallwellensignals an ein Objekt.

7. Diagnostische Ultraschall-Bildgebungsvorrichtung nach Anspruch 6, wobei das Anlegen des Umkehrsignals das Blockieren des Anlegens des Umkehrsignals an das Ausgangsende von dem MUX nach Ausführung des Schaltvorganges zwischen den verschiedenen angelegten Spannungen umfasst.

## Revendications

1. Appareil d'imagerie pour diagnostic par ultrasons, comprenant :

un générateur d'impulsion (310) configuré pour produire une impulsion d'un signal électrique ;
un transducteur (320) configuré pour transformer l'impulsion du signal électrique produit par le générateur d'impulsion (310) en un signal d'onde ultrasonique et pour transmettre le signal d'onde ultrasonique à un objet ;
un multiplexeur, MUX, (330) configuré pour sélectionner un ou plusieurs canaux correspondant à au moins un élément du transducteur (320), pour effectuer une opération de commutation entre différentes tensions appliquées et pour permettre à l'impulsion du signal électrique produite par le générateur d'impulsion (310) d'être transmise par le canal sélectionné ;
**caractérisé en ce que** l'appareil d'imagerie pour diagnostic par ultrasons comprend en outre :

un MUX fictif (340) configuré pour appliquer, à une extrémité de sortie du MUX (330), un signal inverse d'un signal de sortie du MUX (330) en effectuant une opération de commutation inverse qui est opposée à l'opération de commutation par le MUX (330), lorsque le MUX (330) effectue l'opération de commutation entre les différentes tensions appliquées.

2. Appareil d'imagerie pour diagnostic par ultrasons selon la revendication 1, dans lequel le MUX fictif (340) bloque le signal inverse appliqué à l'extrémité de sortie du MUX (330) lors de l'achèvement de l'opération de commutation entre les différentes tensions appliquées.

3. Appareil d'imagerie pour diagnostic par ultrasons selon la revendication 1, dans lequel le MUX (330) effectue l'opération de commutation entre des tensions appliquées de +100 V et -100 V.

4. Appareil d'imagerie pour diagnostic par ultrasons selon la revendication 3, dans lequel le MUX fictif (340) effectue l'opération de commutation inverse de -100 V à +100 V quand l'opération de commutation est effectuée de +100 V à -100 V pour le canal sélectionné.

5. Appareil d'imagerie pour diagnostic par ultrasons selon la revendication 3, dans lequel le MUX fictif (340) effectue l'opération de commutation inverse de +100 V à -100 V quand l'opération de commutation est effectuée de -100 V à +100 V pour le canal sélectionné.

6. Procédé d'imagerie pour diagnostic par ultrasons comprenant :

la production d'une impulsion d'un signal électrique ;
l'exécution, par un multiplexeur, MUX, (330), d'une sélection d'un ou plusieurs canaux correspondant à au moins un élément du transducteur (320) et d'une opération de commutation entre des tensions appliquées différentes ;
l'application, par un MUX fictif (340), à une extrémité de sortie du MUX (330), d'un signal inverse d'un signal de sortie du MUX (330) en effectuant une opération de commutation inverse qui est opposée à l'opération de commutation par le MUX (330) ; et
la transformation de l'impulsion du signal électrique sorti par le canal sélectionné en un signal d'onde ultrasonique et la transmission du signal d'onde ultrasonique à un objet.

7. Procédé d'imagerie pour diagnostic par ultrasons selon la revendication 6, dans lequel l'application du signal inverse comprend le blocage de l'application du signal inverse à l'extrémité de sortie du MUX lors de l'achèvement de l'opération de commutation entre les différentes tensions appliquées.

**FIG. 1**

**FIG. 2**

EP 2 698 113 B1

**FIG. 3**

**FIG. 4**

Removing spike voltage

*330*

SW A     SW B

TX

D     S     S     D

G     G

V_PP(+100V)

V_NN(-100V)

*340*

SW A     SW B

D     S     S     D

G     G

V_PP(+100V)

V_NN(-100V)

# FIG. 5

*FIG. 6*

Start

Generate Pulse of an Electric Signal —S710

Perform Switching Operation between Different
Applied Voltages to a Selected Channel —S720

Apply to Output End of MUX a Reverse Signal in
Opposite Switching Movement to the MUX, in the —S730
Voltage Switching Operation

Convert the Pulse of the Electric Signal Outputted
Through the Selected Channel into Ultrasonic Wave —S740
Signal and Transmit the Same to Object

End

# FIG. 7

Start

Generate Pulse of an Electric Signal — S810

Perform Switching Operation between Different Applied Voltages to a Selected Channel — S820

Apply to Output End of MUX a Reverse Voltage from Reversing a Spike Voltage Due to the Switching Operation, in the Voltage Switching Operation — S830

Convert the Pulse of the Electric Signal Outputted Through the Selected Channel into Ultrasonic Wave Signal and Transmit the Same to Object — S840

End

# FIG. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20020022883 A

### Non-patent literature cited in the description

- **LEQUAN ZHANG et al.** Design of a 64 Channel Receive Beamformer for High Frequency Linear Arrays. *the 2010 IEEE Ultrasonics Symposium (IUS),* 2010, 1968-1971